# EUROPEAN PATENT SPECIFICATION

(11) **EP 3 710 000 B1**
(45) Date of publication and mention of the grant of the patent: **23.04.2025**
(21) Application number: 18821773.1
(22) Date of filing: 16.11.2018
(51) Int. Cl.: A61K 31/444, A61P 1/00, A61P 1/08

(54) **TRADIPITANT FOR USE IN TREATING GASTROPARESIS**
TRADIPITANT ZUR VERWENDUNG BEI DER BEHANDLUNG VON GASTROPARESE
TRADIPITANT DESTINÉ À ÊTRE UTILISÉ DANS LE TRAITEMENT DE LA GASTROPARÉSIE

(30) Priority: 17.11.2017 US 201762587681 P
(43) Date of publication of application: 23.09.2020
(73) Proprietor: Vanda Pharmaceuticals Inc., Washington, DC 20037 (US)
(72) Inventor: POLYMEROPOULOS, Mihael, H., Potomac MD 20854 (US); BIRZNIEKS, Gunther, Chevy Chase MD 20815 (US)
(74) Representative: Appleyard Lees IP LLP
(86) International application number: PCT/US2018/061593
(87) International publication number: WO 2019/099883

(56) References cited:
- WO-A1-2016/141341
- US-B2- 7 381 826
- PASRICHA PANKAJ J ET AL: "Aprepitant Has Mixed Effects on Nausea and Reduces Other Symptoms in Patients With Gastroparesis and Related Disorders", GASTROENTEROLOGY, W.B. SAUNDERS CO, US, vol. 154, no. 1, 28 October 2017 (2017-10-28), pages 65, XP085311721, ISSN: 0016-5085, DOI: 10.1053/J.GASTRO.2017.08.033
- DEEPTI JACOB ET AL: "Effects of NK1 receptors on gastric motor functions and satiation in healthy humans: results from a controlled trial with the NK1 antagonist aprepitant", AMERICAN JOURNAL OF PHYSIOLOGY - GASTROINTESTINAL AND LIVER PHYSIOLOGY, vol. 313, no. 5, 1 November 2017 (2017-11-01), US, pages G505 - G510, XP055561999, ISSN: 0193-1857, DOI: 10.1152/ajpgi.00197.2017

## Description

### BACKGROUND

The application relates generally to use of NK-1 receptor antagonists. More particularly, the application relates the use of the NK-1 antagonist, tradipitant, for treatment of certain tradipitant-responsive diseases and conditions, most specifically gastric motility disorders and conditions in which a therapeutic benefit arises from improvement in gastric motility.

The mammalian tachykinins (neurokinin [NK]) are a family of peptide neurotransmitters that share a common C-terminal sequence. This group includes substance P (SP), neurokinin-A (NKA), and neurokinin-B (NKB). SP exerts its effects by binding to the NK-receptors: SP preferentially binds to the NK-1 receptor and, with lower affinity, to the NK-2 and NK-3 receptors, which preferentially bind NKA and NKB, respectively. The SP receptor is a G-protein-coupled receptor, which, upon binding of SP, activates several second messenger systems, protein kinases, and transcription factors including calcium, inositol triphosphate, p42/44 and p38 stressregulated kinases, and protein kinase C.

SP is the most abundant NK and is involved in the regulation of many physiological processes; for example, in the gut and in the bronchial and vascular systems. In the central nervous system (CNS), SP-containing neurons are distributed in distinct neuronal networks. They are found in the midbrain and basal ganglia, the hypothalamus, the limbic system, and the spinal cord. SP is co-localized with other neurotransmitters and has neuromodulatory effects. Examples of co-localizations are serotonin in the nucleus raphe, dopamine in the midbrain and striatum, gammaaminobutyric acid (GABA) and acetylcholine (ACh) in the cortex, and corticotropinreleasing hormone in the hypothalamus. Examples for direct neuromodulatory effects of SP are the regulation of ACh release in the human cortex and the modulation of noradrenergic neurotransmission in the locus coeruleus.

The NK-1 receptor is as a potential therapeutic target for the treatment of functional gastrointestinal disorders (FGIDs), such as dyspepsia and irritable bowel syndrome (IBS). FGIDs are diagnosed by the presence of recurrent symptoms of abdominal pain or discomfort, and often subjects with IBS generally present with diarrhea or constipation. Current treatments for FGIDs include stress management strategies, dietary modifications, and pharmacotherapy limited to symptomatic treatment. Evidence for the role of neurokinins in abdominal pain has been observed in a double-blind study in which the NK-1 antagonist (CJ-11,974) alleviated the discomfort associated with rectosigmoidal distension in patients with IBS. Both NK-1 and NK-2 receptor antagonists have been investigated for their potential use in alleviating symptoms of food allergy and IBS. The NK-2 receptor antagonist nepadutant (MEN 11420) effectively antagonized the motility-stimulating effect of neurokinin A without affecting basal gastrointestinal motility.

Gastrointestinal (or gastric) motility refers to the coordinated contraction of muscles that digests and propels the contents of the gastrointestinal (GI) tract through the tract. The GI tract includes the esophagus, stomach, small intestine, and colon, each of which has different motility patterns to accomplish different functions. Abnormal motility or sensitivity in any of these sphincter-separated segments of the GI tract may cause characteristic symptoms, which may include abdominal distention, recurrent obstruction, abdominal pain, constipation, nausea, and vomiting. Gastric motility disorders may include, for example, gastroesophageal reflux disease (GERD), intestinal dysmotility, intestinal pseudo-obstruction, small bowel bacterial overgrowth, constipation, outlet obstruction type constipation (pelvic floor dyssynergia), diarrhea, fecal incontinence, Hirschsprung's disease, gastroparesis, and Achalasia.

Gastroparesis is a chronic upper GI disorder characterized by objective evidence of delayed gastric emptying, symptoms associated with gastric retention, and absence of mechanical obstruction. The true prevalence of gastroparesis is unknown, but its US prevalence is estimated to be as high as five million. The two primary etiologies of gastroparesis are Diabetes Mellitus and idiopathic disease, which together represent over 60% of all cases. Nausea is the most commonly reported symptom of gastroparesis, and has been reported in greater than 90% of patients in various studies. Vomiting is the second most commonly reported symptom with reports of incidence varying between 66 and 88 percent. Other commonly reported symptoms of gastroparesis include early satiety, postprandial fullness, abdominal pain, and bloating. Symptoms of gastroparesis may also be present in individuals who do not have delayed gastric emptying. Such individuals may be diagnosed with, e.g., having symptoms of gastroparesis, or having functional dyspepsia or functional nausea. Despite the severity of symptoms and associated distress among gastroparesis patients, there are currently no treatments for the symptoms of gastroparesis approved for chronic use.

Tradipitant is a highly potent, selective, centrally penetrating, and orally active neurokinin-1 (NK-1) receptor antagonist, depicted below as the compound of Formula I 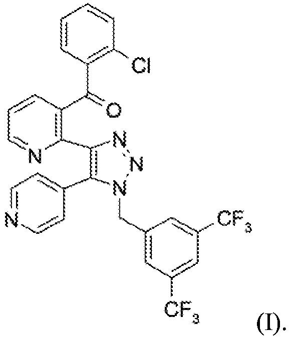 Tradipitant is disclosed in US Pat. 7,320,994, and contains six main structural components: the 3,5-bis-trifluoromethylphenyl moiety, two pyridine rings, the triazol ring, the chlorophenyl ring, and the methanone. Tradipitant is known by the chemical names, 2-[1-[[3,5-bis(trifluoromethyl)phenyl]methyl]-5-(4-pyridinyl)-1H-1,2,3-triazol-4-yl]-3-pyridinyl](2-chlorophenyl)-methanone, and {2-[1-(3,5-bistrifluoromethylbenzyl)-5-pyridin-4-yl-1H-[1,2,3]triazol-4-yl]-pyridin-3-yl}-(2-chlorophenyl)-methanone, and has also been known as LY686017 and as VLY-686. U.S. Patent 7,320,994 describes methods for using compounds, such as tradipitant, for treating a condition associated with an excess of tachykinins, most particularly where the condition associated with an excess of tachykinins is depression and anxiety. The patent further describes the possibility of using compounds, such as tradipitant, in other such diseases, i.e., because these compounds inhibit the physiological effects associated with an excess of tachykinins, the patent describes the usefulness of such compounds in the treatment of numerous other disorders related to tachykinin receptor activation including psychosis, and schizophrenia and other psychotic disorders; neurodegenerative disorders such as dementia, including senile dementia of the Alzheimer's type, Alzheimer's disease, AIDS-associated dementia, and Down syndrome; demyelinating diseases such as multiple sclerosis and amyotrophic lateral sclerosis and other neuropathological disorders, such as peripheral neuropathy, diabetic and chemotherapy-induced neuropathy, and post-herpetic and other neuralgias; acute and chronic obstructive airway diseases such as adult respiratory distress syndrome, bronchopneumonia, bronchospasm, chronic bronchitis, drivercough, and asthma; inflammatory diseases such as inflammatory bowel disease, psoriasis, fibrositis, osteoarthritis, and rheumatoid arthritis; disorders of the musculoskeletal system, such as osteoporosis; allergies such as eczema and rhinitis; hypersensitivity disorders such as poison ivy; ophthalmic diseases such as conjunctivitis, vernal conjunctivitis, and the like; cutaneous diseases such as contact dermatitis, atopic dermatitis, urticaria, and other eczematoid dermatites; addiction disorders such as alcoholism; stress-related somatic disorders; reflex sympathetic dystrophy such as shoulder/hand syndrome; dysthymic disorders; adverse immunological reactions such as rejection of transplanted tissues and disorders related to immune enhancement or suppression such as systemic lupus erythematosis; gastrointestinal disorders or diseases associated with the neuronal control of viscera such as ulcerative colitis, Crohn's disease and irritable bowel syndrome; disorders of bladder function such as bladder detrusor hyper-reflexia and incontinence; atherosclerosis; fibrosin and collagen diseases such as scleroderma and eosinophilic fascioliasis; irritative symptoms of benign prostatic hypertrophy; disorders associated with blood pressure, such as hypertension; or disorders of blood flow caused by vasodilation and vasospastic diseases, such as angina, migraine, and Reynaud's disease; emesis, including chemotherapy-induced nausea and emesis; and pain or nociception, for example, that attributable to or associated with any of the foregoing conditions. Finally, the patent describes such compounds are effective in amounts to be determined that range from 0.001 mg/kg/day to 100 mg/kg/day.

Tradipitant is known to be therapeutically administered through a variety of routes of administration by which it is bioavailable. U.S. Patent 7,320,994 discloses administration of tradipitant by oral and parenteral routes, .e.g., orally, by inhalation, subcutaneously, intramuscularly, intravenously, transdermally, intranasally, rectally, occularly, topically, sublingually, and buccally, with oral administration being generally preferred for treatment.

Crystalline Forms IV and V of tradipitant are disclosed in US Pat. 7,381,826, and a process for preparing crystalline {2-[1-(3,5-bistrifluoromethylbenzyl)-5-pyridin-4-yl-1H-[1,2,3]triazol-4-yl]-pyridin-3-yl}-(2-chlorophenyl)-methanone, Form IV is disclosed in US Pats. 8,772,496 and 9,708,291.

WO2016/141341 discloses a method of treatment with tradipitant, and more particularly a method of treatment of pruritus with tradipitant. Pasricha Pankaj J et al., Gastroenterology 2018; 154: 65 - 67 discloses aprepitant has mixed effects on nausea and reduces other symptoms in patients with gastroparesis and related disorders.

### BRIEF DESCRIPTION OF THE INVENTION

The invention is defined in the appended claims.

Disclosed is the administration of tradipitant to a patient diagnosed with a tradipitant-responsive gastrointestinal (GI) disease or condition, in an amount and at a frequency of administration sufficient to achieve and to maintain a plasma concentration of at least about 100 ng/mL, e.g., about 125 ng/mL or greater, about 150 ng/mL or greater, about 175 ng/mL or greater, about 200 ng/mL or greater, or about 225 ng/mL or greater for the duration of the treatment regimen.

The reference to a patient diagnosed with a tradipitant-responsive gastrointestinal (GI) disease or condition refers herein to a patient diagnosed with one or more of the known diseases or conditions in which tradipitant has been described as being therapeutically useful and specifically includes gastric motility disorders, including gastroparesis, delayed gastric emptying, gastric retention in the absence of mechanical obstruction, or other gastric motility disorders, as well as diseases or conditions manifesting symptoms selected from the group consisting of: nausea, vomiting, early satiety, postprandial fullness, dyspepsia (indigestion), functional dyspepsia, and abdominal pain. In this respect, a tradipitant-responsive GI disease or condition includes disorders in which improvement of gastric motility is therapeutically beneficial. Chemotherapy-induced nausea and vomiting (CINV) is another example of a tradipitant-responsive GI diseases or conditions.

The term "about" as used above and elsewhere herein with respect to concentrations or amount refers to insignificant differences from the quantity that follows. Thus "about 100 ng/mL" is a reference to 100±1 ng/ml.

Disclosed is the administration of tradipitant to a patient diagnosed with a tradipitant-responsive GI disease or condition, the method comprising: orally administering to the patient tradipitant in a solid immediate release form, the solid immediate release form comprising tradipitant and one or more pharmaceutically acceptable excipients, twice daily in an amount of 100 to 400 mg/day, 100 to 300 mg/day, or 100 to 200 mg/day of tradipitant. Such treatment for a patient diagnosed with a tradipitant-responsive GI disease or condition may be for a gastric motility disorder, which may include gastroparesis, delayed gastric emptying, gastric retention in the absence of mechanical obstruction, or other gastric motility disorders, chemotherapy-induced nausea and vomiting (CINV), post-operative nausea and vomiting (PONV), and/or any symptoms selected from the group consisting of: nausea, vomiting, early satiety, postprandial fullness, dyspepsia (indigestion), functional dyspepsia, bloating, and abdominal pain. Treatment may be for diseases or conditions in which improvement of gastric motility is therapeutically beneficial to the patient being treated.

Disclosed is the administration of tradipitant to a patient diagnosed with a tradipitant-responsive GI disease or condition, the method comprising: orally administering to the patient tradipitant once daily in a solid immediate release form, the solid immediate release form comprising tradipitant and one or more pharmaceutically acceptable excipients, in an amount of 150 to 400 mg/day, 150 to 300 mg/day, or 150 to 200 mg/day of tradipitant. Such treatment for a patient diagnosed with a tradipitant-responsive GI disease or condition may be for a gastric motility disorder, which may include gastroparesis, delayed gastric emptying, gastric retention in the absence of mechanical obstruction, or other gastric motility disorders, chemotherapy-induced nausea and vomiting (CINV), post-operative nausea and vomiting (PONV), and/or any symptoms selected from the group consisting of: nausea, vomiting, early satiety, postprandial fullness, dyspepsia (indigestion), functional dyspepsia, bloating, and abdominal pain. Treatment may be for diseases or conditions in which improvement of gastric motility is therapeutically beneficial to the patient being treated.

Also disclosed is the administration of tradipitant to a patient diagnosed with a tradipitant-responsive GI disease or condition, the method comprising: orally administering to the patient tradipitant in an amount of about 170 mg/day of tradipitant. The amount of 170 mg/day may be administered as 85 mg BID, or more specifically as 85 mg Q12H. Such treatment for a patient diagnosed with a tradipitant-responsive GI disease or condition may be for a gastric motility disorder, which may include gastroparesis, delayed gastric emptying, gastric retention in the absence of mechanical obstruction, or other gastric motility disorders, chemotherapy-induced nausea and vomiting (CINV), post-operative nausea and vomiting (PONV), and/or any symptoms selected from the group consisting of: nausea, vomiting, early satiety, postprandial fullness, dyspepsia (indigestion), functional dyspepsia, bloating, and abdominal pain. Treatment may be for diseases or conditions in which improvement of gastric motility is therapeutically beneficial to the patient being treated.

Disclosed is a pharmaceutical composition comprising tradipitant for use in any of the preceding treatments.

Also disclosed is tradipitant for use in the manufacture of a pharmaceutical composition comprising tradipitant for use in any of the preceding treatments.

Also disclosed is a method of treating a gastric motility disorder in an individual, the method comprising: administering to the individual a quantity of tradipitant effective to ameliorate at least one symptom of the gastric motility disorder.

Also disclosed is the administration of tradipitant to an individual suffering from at least one symptom of or associated with gastroparesis, wherein the tradipitant is in an amount sufficient to relieve the at least one symptom. The individual may or may not have a diagnosis of gastroparesis.

These and other aspects, advantages and salient features of the invention will become apparent from the following detailed description, which, when taken in conjunction with the annexed figure(s) disclose embodiments of the invention.

### BRIEF DESCRIPTION OF THE DRAWINGS

FIG. 1 illustrates the effects of tradipitant on NK-1 agonist (GR 73632, 3 pmol, icv)-induced foot-tapping behavior after oral administration.
FIG. 2 illustrates the effects of tradipitant on NK-1 agonist (GR 73632, 3 pmol, icv)-induced foot-tapping behavior after oral administration: comparison with other NK-1 antagonists, MK-869 and CP-122721: dose response.
FIG. 3 illustrates the effects of tradipitant on NK-1 agonist (GR 73632, 3 pmol, icv)-induced foot-tapping behavior after oral administration: comparison with other NK-1 antagonists, MK-869 and CP-122721: time course.
FIG. 4 illustrates the effects of tradipitant on GR73632-induced vocalization in guinea pigs across a concentration range of 0.05 to 10 mg/kg.
FIG. 5 illustrates the duration of activity, i.e. suppression of NK-1 agonist-induced vocalization in guinea pigs, following a 0.1 mg/kg dose of tradipitant.
FIG. 6 illustrates the dose-dependent effects of tradipitant, and the effects of various NK-1 antagonists in the guinea pig vocalization assay.
FIG. 7 shows a bar chart illustrating the median daily nausea score from 64 patients during a 4 week screening period ("Screen") and during an open label extension ("OLE") of the study described in Example 2.2.
FIG. 8 shows a bar chart illustrating the percentage of days that subjects in the study described in Example 2.2 indicate that they are nausea-free, i.e., have a nausea score of 0, during the screening ("Screen") and open label extension ("OLE") periods.

The drawings are intended to depict only typical aspects of the disclosure, and therefore should not be considered as limiting the scope of the disclosure.

### DETAILED DESCRIPTION OF THE INVENTION

At least one embodiment of the present disclosure is described below in reference to its application in connection with the use of tradipitant for the treatment of tradipitant-responsive gastrointestinal (GI) diseases or conditions. Although some embodiments of the disclosure are illustrated relative to specific disorders, e.g., gastroparesis, it is understood that the teachings are equally applicable to other gastric motility disorders, which may include delayed gastric emptying, gastric retention in the absence of mechanical obstruction, or other gastric motility disorders, chemotherapy-induced nausea and vomiting (CINV), post-operative nausea and vomiting (PONV), and/or any symptoms selected from the group consisting of: nausea, vomiting, early satiety, postprandial fullness, dyspepsia (indigestion), functional dyspepsia, bloating, and abdominal pain. In patients suffering from tradipitant-responsive diseases and conditions, patients can be treated by orally administering tradipitant in amounts and at a dosing frequency required to achieve plasma concentrations of at least about 100 ng/mL, e.g., 125 ng/mL or greater, 150 ng/mL or greater, 175 ng/mL or greater, 200 ng/mL or greater, or 225 ng/mL or greater. Such plasma concentration levels can be achieved, e.g., by orally administering to the patient tradipitant in a solid immediate release form comprising one or more pharmaceutically acceptable excipients and tradipitant in an amount of, e.g., 100 to 400 mg/day, 100 to 300 mg/day, 100 to 200 mg/day, or about 85-170 mg/day, which may be administered as, e.g., 50 to 200 mg bid, 50 to 150 mg bid, 50 to 100 mg bid, or about 85 mg bid as described in PCT publication WO 2016/141341 A1. Other particular doses may also be considered part of the invention.

As used herein, the terms "patient," "subject," and "individual" refer to human beings, as well as companion animals (e.g., dogs and cats) and other domesticated animals (e.g., horses, cattle, and sheep). It will be understood that the most preferred patient is a human being.

The disclosure further relates to the treatment of tradipitant-responsive GI diseases or conditions either prophylactically or therapeutically. Further, the terms "treatment" and "treating" are intended to refer to all processes wherein there may be a slowing, interrupting, arresting, controlling, or stopping of the progression of the disorders described herein, and is intended to include prophylactic treatment of such disorders, but does not necessarily indicate a total elimination of all disorder symptoms.

As used herein, the term "effective amount" with reference to the treatment of a tradipitant-responsive GI disease or condition refers to the dose of tradipitant administered to a patient that is effective in treating said disease or condition.

With regard to dosing, "qd" refers to dosing once per day; "bid" dosing typically means dosing once in the morning and once in the evening, generally no less than about 8 hours or more than about 16 hours apart, e.g., 10 to 14 hours or 12 hours (Q12H).

The skilled artisan will appreciate that additional preferred embodiments may be selected by combining the preferred embodiments above, or by reference to the examples given herein.

### EXAMPLES

### 1. Pre-Clinical Studies

Pre-clinical studies demonstrate that tradipitant is a selective neurokinin-1 (NK-1) receptor antagonist. In *in vitro* functional assays, tradipitant potently inhibits NK-1 receptor binding and antagonizes the effects of an NK-1 agonist. In addition, in a panel of 74 additional receptors, enzymes, and ion channels including the NK-2 and NK-3 receptors, no significant activity is observed. By 3 different measures, tradipitant is also a potent centrally active NK-1 antagonist *in vivo.*

### 1.1 Mechanism Studies

Tradipitant inhibits [¹²⁵I] substance P (SP) binding to the NK-1 receptor expressed by IM-9 cells with a Kᵢ of 0.062 nM and inhibits the SP-induced mobilization of intracellular calcium in U373 cells with a K_{b} of 0.095 nM (Table 1).

**Table 1: Affinity of tradipitant for NK-1 Receptors In Vitro**

| **Antagonist** | **IM-9 Human Cell Membrane Binding Kᵢ (nM)** | **Calcium Mobilization in U373 Cells K_{b} (nM)** |
|---|---|---|
| tradipitant | 0.062 ± 0.012 | 0.095 ± 0.025 |
| MK-869 (aprepitant) | 0.14 ± 0.03 | 0.14 ± 0.01 |
| CP-122721 | 0.027 ± 0.01 | 0.034 ± 0.009 |

These potencies are similar to the observed potencies of NK-1 antagonists MK-869 (aprepitant) and CP-122721. In addition, tradipitant is evaluated in a panel of 74 other receptors, enzymes, and ion channels. At a test concentration of 1 µM, tradipitant does not exhibit any inhibition of binding greater than 50%. At the NK-2 and NK-3 receptors, the compound produces no significant inhibition. Therefore, tradipitant is a highly selective NK-1 antagonist *in vitro.*

Several of the major metabolites of tradipitant are synthesized and their affinity for the NK-1 receptor tested in the binding assay (Table 2). These metabolites have high affinity for the NK-1 receptor.

**Table 2: Affinity of tradipitant Metabolites for NK-1 Receptors In Vitro**

| **Metabolite** | **Designation** | **IM-9 Human Cell Membrane Binding Kᵢ (nM)** |
|---|---|---|
| LSN2081070 | M2 (Racemic) | 0.09 (n=1) |
| LSN2107355 | M2 (S-enantiomer) | 0.08 (n=1) |
| LSN2107357 | M2 (R-enantiomer) | 0.94 (n=1) |
| LSN2195411 | M3 | 0.03 (n=1) |
| LSN2195413 | M4 (Racemic) | (n=1) |

### 1.2 Efficacy Models

Several *in vivo* models are used to evaluate the brain NK-1 receptor occupancy and efficacy of tradipitant.

### 1.2.1 Effects of Tradipitant on Centrally Administered NK-1 Agonist-Induced Foot-Tapping Behavior in Gerbils

### Introduction

Differences in species selectivity of NK-1 receptors pose challenges to characterization of NK-1 receptor antagonists in vivo. Gerbil NK-1 receptors are shown to be similar to those in humans (Gitter et al. 1991, Beresford et al. 1991). Gerbils exhibit a characteristic stereotypic foot-tapping behavior in response to distress, fear or aversive stimuli (Routtenberg and Kramis 1967; Holman 1985; Ballard et al. 2001). Intracerebroventricular (icv) administration of substance P or selective NK-1 receptor agonists such as GR 73632, produces rapid rhythmic tapping of the hind feet (Graham et al. 1993) lasting approximately 5 minutes, which can be inhibited by systemic administration of brain penetrating antagonists of the NK-1 receptor (Bristow and Young 1994; Rupnaik and Williams 1994). This response is selective for NK-1 agonists, since selective NK-2 and NK-3 agonists do not elicit a similar response (Graham et al. 1993; Li and Iyengar unpublished). This behavioral response is further characterized and modified to enable identification and optimization, in vivo, of potent NK-1 receptor antagonists including tradipitant.

### Methods

Male Mongolian gerbils (Harlan Sprague Dawley, Indianapolis, IN) weighing 26 to 40 grams are administered the selective neurokinin-1 receptor agonist GR73632 (3 pmol) via direct, vertical, free-hand intracerebroventricular (icv) injection to a depth of 4.5 mm below bregma with a cuffed 27-gauge needle attached to a 50 µl Hamilton syringe. Immediately after injection, animals are placed individually into isolated chambers with pressure-sensitive velocimeter platform floors (San Diego Instruments acoustic startle apparatus) that detect and quantify vibration. The San Diego Instruments "SR" DOS-based computer program is used on a PC to record the number of foot-taps over the following 6 to 10 minutes, beginning 30 seconds after the floor was lightly tapped. Raw data is converted with a Microsoft Excel macro that determines the number of events over threshold (125) in each 250-millisecond time bin over the 5.5 minutes following onset of observation. The total number and average intensity of events over the duration is determined. Total number of taps is analyzed with one-way ANOVA and post-hoc Dunnett's test using JMP statistical software.

A dose-response curve (with doses of 0.3, 1, 3 and 10 pmols, icv) is initially generated with the NK-1 agonist GR73632. Maximal foot-tapping behavior is achieved with 3 and 10 pmol doses. The 3 pmol dose is subsequently chosen as the dose of choice for antagonism experiments.

NK-1 antagonists are tested for their ability to attenuate GR73632-induced foot tapping. NK-1 antagonists are administered (po) via oral feeding tube at doses and time points specified in each experiment. All animals receive only one dose of NK-1 antagonists in all tests.

### ED₅₀ Determinations/Dose-Response Tests

NK-1 antagonists are administered at multiple doses (at least 3; one dose per animal) and response to GR73632 is measured.

### Duration of Action Tests

NK-1 receptor antagonists including tradipitant, MK-869 (aprepitant), and CP-122721 are administered at multiple pre-treatment times (one administration per animal) including at 0.5, 1, 2, 4, 7, 16, and 24 hours prior to injection of GR73632 injection (Peninsula Labs, CA) dissolved in saline. Tradipitant is dissolved in 1% CMC/0.5% SLS/0.085% PVP vehicle. CP-122721 and MK-869 (Aprepitant) are synthesized at Lilly Laboratories and dissolved in 10% ethanol/emulphor and 1% CMC/0.5% SLS/0.085% PVP respectively.

### Results

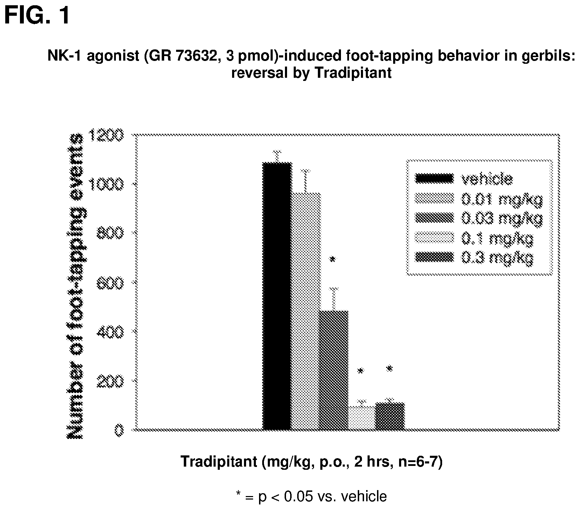

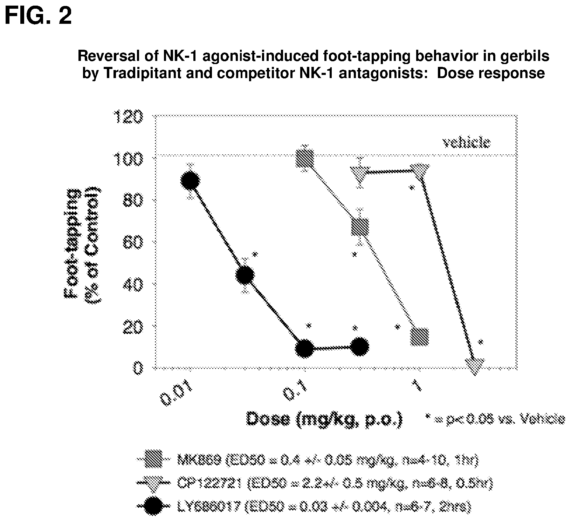

Orally administered tradipitant (0.01, 0.03, 0.1, 0.3 mg/kg, po) potently inhibits NK-1 agonist (GR 73632, 3 pmol, icv)-induced foot-tapping behavior in gerbils, in a dose-dependent manner (FIG. 1), with an ED₅₀ of 0.03 mg/kg. In FIG. 1, y-axis data is expressed in number of foot-tapping events in five minutes. The efficacy of tradipitant in this assay is compared to that of other NK-1 antagonists, MK-869 and CP-122721 (FIG. 2). Tradipitant is found to be more potent than MK-869 (Merck, ED₅₀ = 0.42 mg/kg, and CP-122721 (Pfizer, ED₅₀ =2.2 mg/kg). Tradipitant (0.01, 0.03, 0.1, 0.3 mg/kg, po) inhibits NK-1 agonist foot-tapping behavior with an ED₅₀ of 0.03 + 0.004 mg/kg. In comparison, MK-869 inhibits foot-tapping behavior with an ED₅₀ of 0.4 + 0.05 mg/kg, and CP 122721 inhibits behavior at a dose of 2.2 + 0.5 mg/kg (mean ± SE). Data in FIG. 2 are expressed as percent control of vehicle (vehicle response to 3 pmol GR73632).

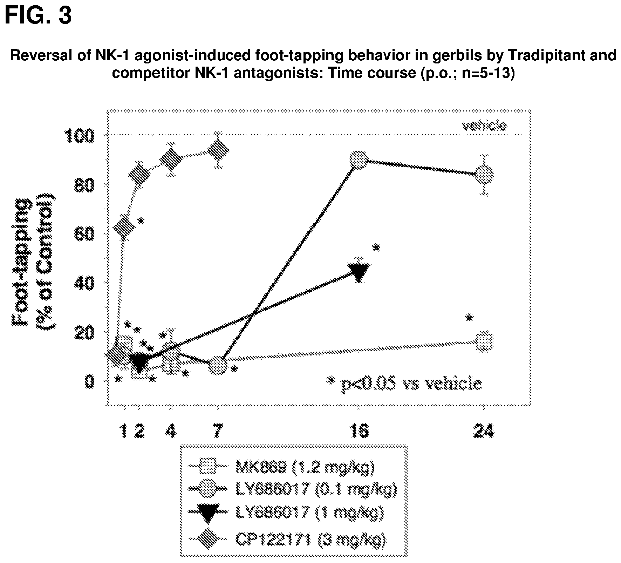

Tradipitant (0.1 mg/kg, po) is found to significantly inhibit NK-1 agonist induced foot-tapping behavior up to 7 hours after administration (FIG. 3) but the effect was significantly diminished by 16 hours after administration at this dose. However, at a higher dose of 1 mg/kg (FIG. 3), tradipitant significantly reverses foot-tapping behavior (greater than 50% inhibition of foot-tapping behavior) up to 16 hours after administration. The duration of effects of tradipitant (FIG. 3) is longer than that of CP-122721 (3 mg/kg), which inhibits foot-tapping behavior up to 2 hours after administration, while MK-869 (1.2 mg/kg, po) significantly inhibits foot-tapping behavior up to 24 hours after administration. Mean ± SE *p<.05 compared to vehicle. Data are expressed as percent control of vehicle (vehicle response to 3 pmol GR73632).

### Discussion

The effects of tradipitant on NK-1 agonist-induced foot-tapping behavior in gerbils suggest that tradipitant is a very potent, centrally acting NK-1 receptor antagonist *in vivo* in the gerbil with a relatively long duration of action.

### 1.2.2: Emetic Challenge Study in Beagle Dogs with Tradipitant

### Introduction

Tradipitant is a potent and selective NK-1 receptor antagonist with demonstrated efficacy in animal models of distress. Five male dogs are administered a single oral dose of 3 mg/kg MK-869 (a positive control), or tradipitant at 0.3, 1.0, and 3.0 mg/kg in a Latin-square design. An intravenous injection of 0.1 mg/kg apomorphine, a known emetic, is given alone, or 2 hours after administration of tradipitant or MK-869. Each animal is administered a different dose on a particular dosing day, so that each dose of tradipitant, MK-869, and apomorphine alone is represented. Over the five (5) weeks of the study, each animal receives each of the treatments, but only one per week. The purpose of this study is to determine whether tradipitant suppresses apomorphine-induced emesis.

The low dose of tradipitant is 10 times the ED₅₀ in the gerbil foot-tapping model of NK-1 receptor antagonism (Example 1.2.1). The high dose is 100 times this efficacious dose, and is also the dose of MK-869 that was determined to be efficacious against apomorphine-induced emesis in the dog (Gidda, 2003). The mid dose of tradipitant is the approximate half-log interval between the low and high doses.

The oral route of administration is selected for tradipitant because this is the route proposed, or currently used, clinically. The intravenous route is typically used for experimental apomorphine administration. The beagle dog is considered an effective species for demonstration of antagonism of apomorphine-induced emesis (Gidda, 2003).

### Methods

A single oral dose of 0, 0.3, 1.0, or 3.0 mg tradipitant/kg, or 3.0 mg MK-869/kg, is administered to each male dog once a week in gelatin capsules. All animals are dosed over a period of five (5) weeks, with each dog receiving one of five different treatments on each day of dosing. A dose of 0.1 mg apomorphine/kg is administered by intravenous injection approximately 2 hours after each administration of tradipitant or MK-869. In cases where apomorphine was administered alone, without prior treatment with tradipitant or MK-869, apomorphine is given at approximately the same time as when given in combination with tradipitant or MK-869.

All dogs are fasted overnight prior to each treatment day and are then fed approximately one (1) hour after oral dosing (approximately one hour prior to administration of apomorphine). Individual doses are adjusted weekly for changes in body weight.

The dose regimen consists of a 5x5 Latin square design, in which each subject receives 1 dose or dose combination/week (6 day washout) as shown in Table 3 below.

**Table 3: Latin Square Design**

| **Study week** | **1** | **2** | **3** | **4** | **5** |
|---|---|---|---|---|---|
| **Dog 1** | APO + 0.3 mg/kg tradipitant | APO + MK-869 | APO + 3 mg/kg tradipitant | APO | APO + 1 mg/kg tradipitant |
| **Dog 2** | APO + MK-869 | APO + 1 mg/kg tradipitant | APO | APO + 0.3 mg/kg tradipitant | APO + 3 mg/kg tradipitant |
| **Dog 3** | APO + 3 mg/kg tradipitant | APO | APO + MK-869 | APO + 1 mg/kg tradipitant | APO + 0.3 mg/kg tradipitant |
| **Dog 4** | APO | APO + 0.3 mg/kg tradipitant | APO 1 mg/kg tradipitant | APO + 3 mg/kg tradipitant | APO + MK-869 |
| **Dog 5** | APO 1 mg/kg tradipitant | APO + 3 mg/kg tradipitant | APO + 0.3 mg/kg tradipitant | APO + MK-869 | APO |

The number of emetic episodes is recorded for approximately one hour following the injection of apomorphine, and plasma concentrations at anticipated Tmax of tradipitant (2 hours post-dosing) are evaluated.

### Results

Individual, mean, and standard deviation values for the 2 hour plasma concentrations of tradipitant are shown in the following table. All animals administered tradipitant have measurable levels at 2 hours post-dose. In general, plasma concentrations at 2 hours post-dose increase with increasing dose in a subproportional manner. As observed in other studies in dogs, the exposure to tradipitant is variable between animals. Individual animal data reveal no relationship between plasma concentrations and week of administration.

**Table 4: Plasma concentrations of tradipitant (ng/ml)**

| **Administered Dose** | **0.3 mg/kg** | **1.0 mg/kg** | **3.0 mg/kg** |
|---|---|---|---|
| | Concentration of tradipitant (ng/ml) | Concentration of tradipitant (ng/ml) | Concentration of tradipitant (ng/ml) |
| **Dog 1** | 51.20 | 175.58 | 122.73 |
| **Dog 2** | 41.33 | 86.49 | 256.58 |
| **Dog 3** | 90.93 | 240.84 | 316.20 |
| **Dog 4** | 83.38 | 100.97 | 682.91 |
| **Dog 5** | 22.59 | 61.56 | 119.79 |
| **Mean (SD)** | 57.89 (28.75) | 133.09 (73.71) | 299.64 (230.58) |

Emesis occurs after each treatment, with the largest incidence of emesis occurring in the apomorphine alone group. One dog (Dog 3) has a single episode of emesis at each dose of tradipitant and MK-869; this dog also has the greatest number of emetic episodes with apomorphine alone (12). No emesis occurs in the remaining four (4) dogs at any dose of tradipitant or MK-869. These dogs have an average of four (4) emetic episodes with apomorphine alone. The antiemetic effect of MK-869 supports the validity of this model.

**Table 5: Emetic episodes by treatment group**

| **Test article*** | **Dose level (mg/kg)** | **Total No.Emetic Episodes** |
|---|---|---|
| APO (control) | 0 | 28 |
| MK-869 | 3.0 | 1** |
| tradipitant | 0.3 | 1** |
| tradipitant | 1.0 | 1** |
| tradipitant | 3.0 | 1** |

| | | |
|---|---|---|
| *Apomorphine is administered as a challenge dose to all groups. **All episodes occur in same dog (Dog 3). | | |

Results of this study indicate that tradipitant is effective against apomorphine-induced emesis at each dose tested (0.3, 1.0, and 3.0 mg/kg).

### 1.2.3 Tradipitant Inhibits Substance P-Induced Vocalization in Guinea Pigs

### Introduction

When introduced into the brain, the NK-1 receptor agonist, substance P (SP) elicits distress vocalizations in the guinea pig that can be inhibited by NK-1 antagonists (Kramer et al. 1998; Rupniak et al. 2000). This behavioral assay is used to demonstrate potency and CNS penetration of NK-1 antagonists in the guinea pig, a species that has receptor affinity for NK-1 antagonists that is similar to humans.

### Methods

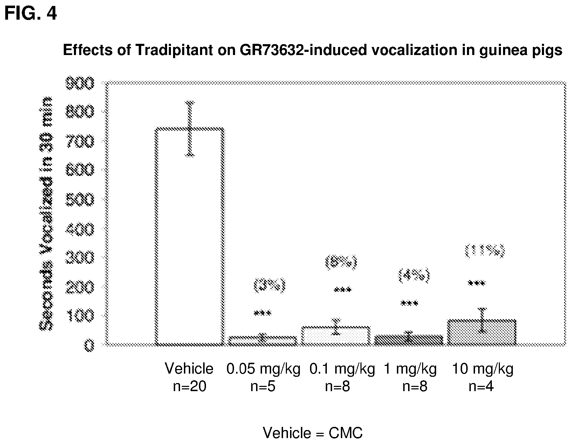

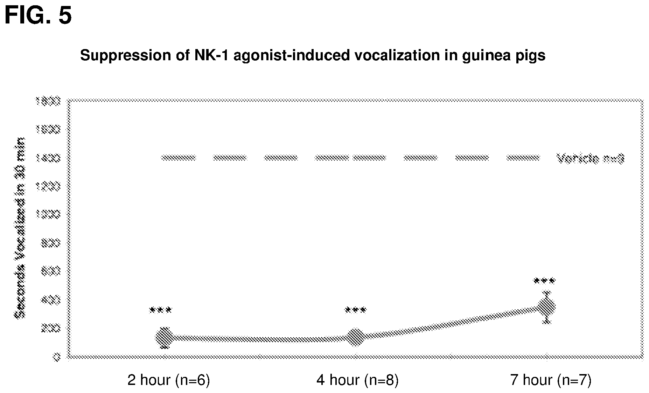

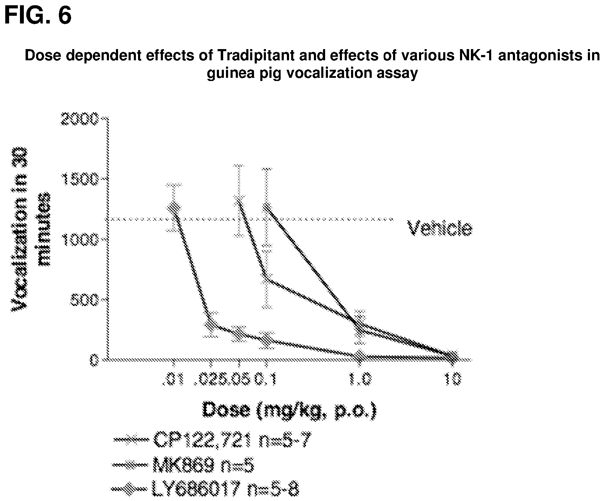

Male Dunkin/Hartley guinea pigs (200 to 250 grams) are orally administered either vehicle or an NK-1 antagonist. Approximately 45 minutes later (for dose response studies), the animals are anesthetized and 0.1 nmol of GR73632 (SP analog) in a vehicle volume of 5 µl is injected into the cerebral ventricle at the intersection of bregma and the midline of the skull. Animals are placed in a dark testing chamber located inside of a sound attenuation cubicle and vocalizations are recorded for 30 minutes following recovery from anesthesia. The time spent vocalizing is quantified for each animal. In the duration of action study (FIG. 5), 0.1 mg/kg of tradipitant or vehicle solution is administered orally and then 2, 4, or 7 hours later, 0.1 nmol of GR73632 is administered into the cerebral ventricle as described above. Vocalizations are recorded and quantified as indicated above. Vehicle solutions are either CMC (FIG. 4 data) or a 10% ethanol/emulphor, 10% propylene glycol solution (FIGS. 5 and 6). Data is analyzed using one-tailed t-tests.

### Results

Data summarized in FIG. 4 indicate that oral administration of tradipitant produces significant inhibition of agonist-induced vocalization at doses of 10 mg/kg (p < 0.001), 1.0 mg/kg (p < 0.001), 0.1 mg/kg (p < 0.001), and 0.05 mg/kg (p < 0.001). Activity of tradipitant does not wane at the lower doses, indicating that even lower doses would be required to produce a dose response function. In FIG. 4, parenthetical numbers indicate percent of control response.

A duration of activity study indicates that the effect of a single dose of 0.1 mg/kg tradipitant is significantly active (FIG. 5) in suppressing agonist-induced vocalization at 7 hours following oral administration of the antagonist compound.

In a second dose-response study (FIG. 6), potencies of various NK-1 antagonists in the guinea pig vocalization assay are compared. Vehicle is an ethanol/ emulphor solution; vehicle groups are n=5-14 per compound. Lower concentrations of tradipitant are tested and these data are presented with data obtained using Merck (MK-869), and Pfizer (CP-122721). All NK-1 antagonists tested produce significant inhibition of vocalization at 1 mg/kg (p < 0.001). Only tradipitant retains significant inhibitory activity at and below 0.1 mg/kg, as well as at lower doses of 0.05 and 0.025 mg/kg (p < 0.001). The minimum effective dose of tradipitant is found to be 0.025 mg/kg, which produces highly significant (p < 0.001) inhibition of vocalization compared to controls.

### Discussion

Tradipitant significantly inhibits NK-1 agonist-induced vocalization in guinea pigs, indicating that this compound is an orally available and brain penetrant NK-1 antagonist. The minimum effective dose (MED) that produces this effect is 0.025 mg/kg. The compound, administered orally, is shown to have a duration of activity that exceeds 7 hours. In this experimental paradigm, tradipitant is substantially more potent than MK-869 and CP-122721.

### 1.2.4 Occupancy of NK-1 receptors

A tracer NK-1 antagonist compound (GR205171) is used to evaluate the ability of other NK-1 antagonists to occupy the brain NK-1 receptors. In these studies, the test compounds are administered orally and the tracer compound administered intravenously afterward. The occupancy of the NK-1 receptors is evaluated by quantitating the amount of the tracer compound bound to the brain NK-1 receptors after increasing doses of the test compounds. Using this paradigm, tradipitant has an estimated ED₅₀ of 0.04 mg/kg p.o. and is substantially more potent than the other antagonists evaluated.

### 2. Clinical Studies.

### 2.1 Gastrointestinal motility

A single-center, randomized, double-blind, placebo-controlled study is conducted to investigate the effect of tradipitant on small bowel transit time. A total of 15 healthy subjects, 12 men and 3 women, between the ages of 19 and 63 years are enrolled in the study and receive at least 1 dose of study medication. A total of 13 subjects complete the study. Subjects are randomized to receive 20 mg of tradipitant, 200 mg of tradipitant, or placebo as a single oral dose within each of 3 periods, coadministered with a capsule radiolabeled with a maximum of 1MBq ¹¹¹In. Four hours post-dose, all subjects also receive a second capsule radiolabeled with a maximum of 4MBq ^{99m}Tc. Each subject receives all 3 doses during the study. For all dosing regimens, *in vivo* gamma scintigraphic studies are performed at predetermined intervals, and the following scintigraphic parameters are analyzed: onset and completion of gastric emptying, onset and completion of colon arrival, initiation and completion of small bowel transit, and initial and complete disintegration of the capsule (anatomical location and time).

A statistically significant effect of tradipitant on small bowel transit time is observed in the study. No effect on gastric emptying is observed in this study. However, the study is underpowered with respect to this parameter.

### 2.2 Gastroparesis

In a phase II study of tradipitant in patients with gastroparesis, participants initially enter 4 week screening period prior to treatment with tradipitant, during which they answer a daily symptom questionnaire rating their nausea severity on a scale of 0 to 5, in which 0 represents no nausea, 1 represents very mild nausea, 2 represents mild nausea, 3 represents moderate nausea, 4 represents severe nausea, and 5 represents very severe nausea. After the 4 week screening period, patients enter the double blind randomized portion of the study, in which they receive either tradipitant 85 mg bid, or placebo. After 4 weeks of blinded study drug treatment, patients have the opportunity to enter an additional 8 week open label extension, during which each subject receives tradipitant 85 mg bid. 64 subjects participate in at least one week of the open label tradipitant dosing portion of the study. The average daily diarized nausea scores are reported for subjects during the 4 week screening period and during the up to 8 week open label extension.

### Results

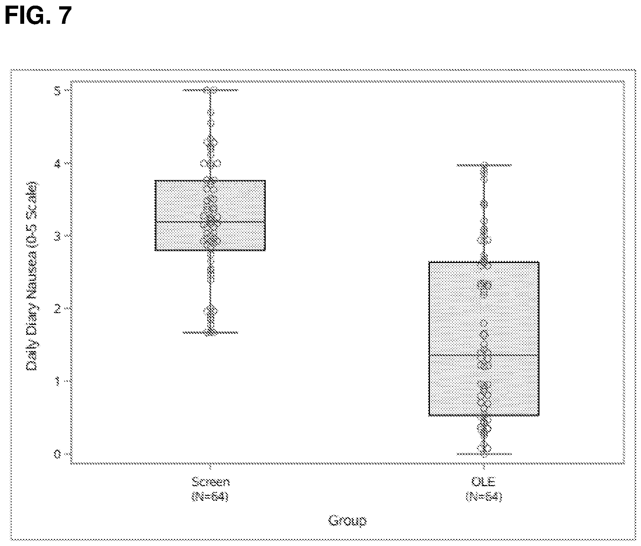

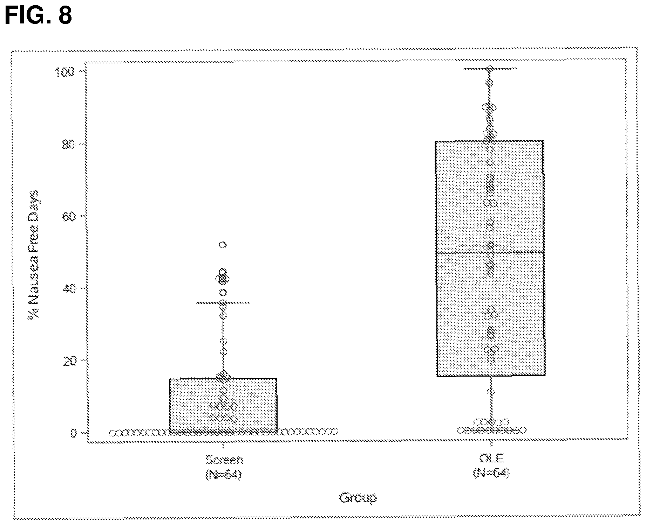

During the screening period, i.e. prior to randomization, subjects report a mean daily nausea score over the 4 week period of about 3.20 (± std. dev. of 0.83) on the 0 to 5 scale, i.e. greater than "moderate nausea." During the open label portion of the study, during which the same 64 patients receive 85 mg bid tradipitant for a duration of 1 to 8 weeks, the same patients report a mean daily nausea score of about 1.63 (± std. dev. of 1.17) on the 0 to 5 scale. FIG. 7 illustrates the median daily nausea scores for the 64 subjects in the screening period (3.19) and open label extension period (1.35) respectively. Additionally, during the screening period prior to randomization, subjects report a mean 9.39% days (± std. dev. of 14.98) nausea-free, while during the open label extension, subjects report a mean 45.91% of days (± std. dev. of 33.64) nausea-free. FIG. 8 illustrates the median percentage of nausea-free days during the screening period (0%) and during the open label extension (49.11%).

### Conclusions

The results described above and illustrated in FIGS. 7 and 8 demonstrate that in subjects suffering from gastroparesis, treatment with 85 mg bid tradipitant significantly improves both subjects' average daily nausea scores and experiences of nausea-free days. Compared to pre-treatment, administration of 85 mg bid tradipitant results in subjects reporting average daily nausea scores that are reduced by approximately one half, and average percentage of nausea-free days that are doubled or greater.

### EMBODIMENTS

In addition to other illustrative embodiments, this disclosure can be seen to comprise one or more of the following illustrative embodiments:
1. A method of treatment of a patient diagnosed with a tradipitant-responsive gastrointestinal (GI) disease or condition comprising: administering to said patient tradipitant, in an amount and at a frequency of administration sufficient to achieve and to maintain a plasma concentration of at least about 100 ng/mL, about 125 ng/mL, about 150 ng/mL, about 175 ng/mL, about 200 ng/mL, or about 225 ng/mL for the duration of the treatment regimen.
2. The method of embodiment 1, wherein the tradipitant is orally administered in a solid immediate release form comprising tradipitant and one or more pharmaceutically acceptable excipients.
3. The method of embodiment 1, wherein the tradipitant is orally administered in a solid controlled release form comprising tradipitant and one or more pharmaceutically acceptable excipients.
4. A method of treating a patient diagnosed with a tradipitant-responsive gastrointestinal (GI) disease or condition, the method comprising: orally administering to the patient a solid immediate release form comprising tradipitant and one or more pharmaceutically acceptable excipients, twice daily in an amount of 100 to 400 mg/day, 100 to 300 mg/day, or 100 to 200 mg/day of tradipitant.
5. A method of treating a patient diagnosed with a tradipitant-responsive gastrointestinal (GI) disease or condition, the method comprising: orally administering to said patient tradipitant once daily, in a solid immediate release form, said solid immediate release form comprising tradipitant and one or more pharmaceutically acceptable excipients, in an amount of 150 to 400 mg/day, 150 to 300 mg/day, or 150 to 200 mg/day of tradipitant.
6. A method of treating a patient diagnosed with a tradipitant-responsive gastrointestinal (GI) disease or condition, the method comprising: orally administering to said patient tradipitant in an amount of about 170 mg/day.
7. The method of embodiment 6, wherein the amount of 170 mg/day is 85 mg BID.
8. The method of embodiment 6, wherein the amount of 170 mg/day is 85 mg Q12H.
9. The method of any of embodiments 1-8, wherein the tradipitant-responsive GI disease or condition is gastroparesis.
10. The method of any of embodiment 1-8, wherein the tradipitant-responsive GI disease or condition is a gastric motility disorder.
11. The method of any of embodiments 1-8, wherein the tradipitant-responsive GI disease or condition is delayed gastric emptying.
12. The method of any of embodiments 1-8, wherein the tradipitant-responsive GI disease or condition is gastric retention in the absence of mechanical obstruction.
13. The method of any of embodiments 1-8, wherein the tradipitant-responsive GI disease or condition is a disorder in which improvement of gastric motility is therapeutically beneficial.
14. The method of any of embodiments 1-8, wherein the patient is being treated for at least one symptom selected from the group consisting of: nausea, vomiting, early satiety, postprandial fullness, dyspepsia, functional dyspepsia, bloating, and abdominal pain.
15. The method of any of embodiments 1-8, wherein the tradipitant-responsive GI disease or condition is chemotherapy-induced nausea and vomiting.
16. The method of any of embodiment 1-8, wherein the tradipitant-responsive GI disease or condition is post-operative nausea and vomiting.
17. Tradipitant for use in any of the preceding methods of treatment.
18. A pharmaceutical composition comprising tradipitant for use in any of the preceding methods.
19. Tradipitant for use in the manufacture of a pharmaceutical composition comprising tradipitant for use in any of the preceding methods.
20. A method of treating a gastric motility disorder in an individual, the method comprising: administering to the individual a quantity of tradipitant effective to ameliorate at least one symptom of the gastric motility disorder that is 170 mg/day.
21. The method, the tradipitant, or the pharmaceutical composition of any of the preceding embodiments, wherein the tradipitant is in crystalline Form IV or Form V.
22. A method of treatment comprising: administering tradipitant to an individual suffering from at least one symptom of gastroparesis, wherein the tradipitant is in an amount sufficient to relieve the at least one symptom of gastroparesis that is 170 mg/day.
23. The method of embodiment 22, wherein the symptom of gastroparesis is delayed gastric emptying.
24. The method of embodiment 22, wherein the symptom of gastroparesis is nausea.
25. The method of embodiment 22, wherein the symptom of gastroparesis is vomiting.
26. The method of embodiment 22, wherein the symptom of gastroparesis is early satiety.
27. The method of embodiment 22, wherein the symptom of gastroparesis is postprandial fullness.
28. The method of embodiment 22, wherein the symptom of gastroparesis is abdominal pain.
29. The method of embodiment 22, wherein the symptom of gastroparesis is bloating.
30. The method of embodiment 22, wherein the individual has a diagnosis of gastroparesis.
31. The method of embodiment 22, wherein the individual does not have a diagnosis of gastroparesis.
32. The method of embodiment 22, wherein the individual has a diagnosis of functional dyspepsia.
33. The method of embodiment 22, wherein the individual has a diagnosis of functional nausea.

## Claims

1. Tradipitant for use in the treatment of a human individual suffering from gastroparesis, comprising:
orally administering to said human individual a solid immediate release form comprising one or more pharmaceutically acceptable excipients and tradipitant at a dose of 100-400 mg/day.

2. Tradipitant for use according to claim 1, wherein the dose of tradipitant is 150 to 400 mg/day. given once daily. Z

3. Tradipitant for use according to claim 2, wherein the dose of tradipitant is 150 to 300 mg/day. given once daily.

4. Tradipitant for use according to claim 3, wherein the dose of tradipitant is 150 to 200 mg/day. given once daily.

5. Tradipitant for use according to claim 1, wherein the dose of tradipitant is 100 to 300 mg/day.

6. Tradipitant for use according to claim 5, wherein the dose of tradipitant is 100 to 200 mg/day.

7. Tradipitant for use according to claim 1, wherein the dose of tradipitant is about 170 mg/day.

8. Tradipitant for use according to any of claims 1 or 5-7, wherein the tradipitant is administered twice daily.

9. Tradipitant for use according to claim 1, wherein the dose of tradipitant is 170 mg/day given as 85 mg twice daily (BID).

10. Tradipitant for use according to any of claims 1-9, wherein the human individual is experiencing at least one symptom selected from the group consisting of: nausea, vomiting, early satiety, postprandial fullness, dyspepsia, functional dyspepsia, bloating, and abdominal pain.

11. Tradipitant for use according to any of claims 1-9, wherein the human individual is suffering from a symptom selected from: delayed gastric emptying, nausea, vomiting, early satiety, postprandial fullness, abdominal pain, and bloating.

12. Tradipitant for use according to any of claims 1-9, wherein the human individual is experiencing nausea or vomiting.

13. A pharmaceutical composition comprising tradipitant and one or more pharmaceutically acceptable excipients, for use according to any of claims 1-12.

## Patentansprüche

1. Tradipitant zur Verwendung bei der Behandlung eines menschlichen Individuums, das an Gastroparese leidet, umfassend:
orales Verabreichen einer festen Form zur sofortigen Freisetzung, die einen oder mehrere pharmazeutisch unbedenkliche Exzipienten und Tradipitant in einer Dosis von 100-400 mg/Tag umfasst, an das menschliche Individuum.

2. Tradipitant zur Verwendung nach Anspruch 1, wobei es sich bei der Tradipitant-Dosis um 150 bis 400 mg/Tag, einmal täglich gegeben, handelt.

3. Tradipitant zur Verwendung nach Anspruch 2, wobei es sich bei der Tradipitant-Dosis um 150 bis 300 mg/Tag, einmal täglich gegeben, handelt.

4. Tradipitant zur Verwendung nach Anspruch 3, wobei es sich bei der Tradipitant-Dosis um 150 bis 200 mg/Tag, einmal täglich gegeben, handelt.

5. Tradipitant zur Verwendung nach Anspruch 1, wobei die Tradipitant-Dosis bei 100 bis 300 mg/Tag liegt.

6. Tradipitant zur Verwendung nach Anspruch 5, wobei die Tradipitant-Dosis bei 100 bis 200 mg/Tag liegt.

7. Tradipitant zur Verwendung nach Anspruch 1, wobei die Dosis von Tradipitant etwa 170 mg/Tag beträgt.

8. Tradipitant zur Verwendung nach einem der Ansprüche 1 oder 5-7, wobei das Tradipitant zweimal täglich verabreicht wird.

9. Tradipitant zur Verwendung nach Anspruch 1, wobei es sich bei der Tradipitant-Dosis um 170 mg/Tag, als zweimal täglich (BID) 85 mg gegeben, handelt.

10. Tradipitant zur Verwendung nach einem der Ansprüche 1-9, wobei bei dem menschlichen Individuum wenigstens ein Symptom auftritt, das aus der Gruppe bestehend aus Übelkeit, Erbrechen, frühzeitigem Sattsein, postprandialem Völlegefühl, Dyspepsie, funktioneller Dyspepsie, Blähungen und Bauchschmerzen ausgewählt ist.

11. Tradipitant zur Verwendung nach einem der Ansprüche 1-9, wobei das menschliche Individuum an einem Symptom leidet, das aus verzögerter Magenentleerung, Übelkeit, Erbrechen, frühzeitigem Sattsein, postprandialem Völlegefühl, Bauchschmerzen und Blähungen ausgewählt ist.

12. Tradipitant zur Verwendung nach einem der Ansprüche 1-9, wobei bei dem menschlichen Individuum Übelkeit oder Erbrechen auftritt.

13. Pharmazeutische Zusammensetzung, umfassend Tradipitant und einen oder mehrere pharmazeutisch unbedenkliche Exzipienten, zur Verwendung nach einem der Ansprüche 1-12.

## Revendications

1. Tradipitant pour une utilisation dans le traitement d'un individu humain souffrant de gastroparésie, comprenant :
l'administration par voie orale audit individu humain d'une forme solide à libération immédiate comprenant un ou plusieurs excipients pharmaceutiquement acceptables et du tradipitant à une dose de 100-400 mg/jour.

2. Tradipitant pour une utilisation selon la revendication 1, dans lequel la dose de tradipitant est de 150 à 400 mg/jour donnée une fois par jour.

3. Tradipitant pour une utilisation selon la revendication 2, dans lequel la dose de tradipitant est de 150 à 300 mg/jour donnée une fois par jour.

4. Tradipitant pour une utilisation selon la revendication 3, dans lequel la dose de tradipitant est de 150 à 200 mg/jour donnée une fois par jour.

5. Tradipitant pour une utilisation selon la revendication 1, dans lequel la dose de tradipitant est de 100 à 300 mg/jour.

6. Tradipitant pour une utilisation selon la revendication 5, dans lequel la dose de tradipitant est de 100 à 200 mg/jour.

7. Tradipitant pour une utilisation selon la revendication 1, dans lequel la dose de tradipitant est d'environ 170 mg/jour.

8. Tradipitant pour une utilisation selon l'une quelconque des revendications 1 ou 5 à 7, dans lequel le tradipitant est administré deux fois par jour.

9. Tradipitant pour une utilisation selon la revendication 1, dans lequel la dose de tradipitant est de 170 mg/jour donnée sous la forme de 85 mg deux fois par jour (deux fois par jour).

10. Tradipitant pour une utilisation selon l'une quelconque des revendications 1 à 9, dans lequel l'individu humain éprouve au moins un symptôme choisi dans le groupe constitué par : nausées, vomissements, satiété précoce, plénitude postprandiale, dyspepsie, dyspepsie fonctionnelle, ballonnements et douleurs abdominales.

11. Tradipitant pour une utilisation selon l'une quelconque des revendications 1 à 9, dans lequel l'individu humain souffre d'un symptôme choisi parmi : une vidange gastrique retardée, des nausées, des vomissements, une satiété précoce, une plénitude postprandiale, des douleurs abdominales et des ballonnements.

12. Tradipitant pour une utilisation selon l'une quelconque des revendications 1 à 9, dans lequel l'individu humain éprouve des nausées ou des vomissements.

13. Composition pharmaceutique comprenant du tradipitant et un ou plusieurs excipients pharmaceutiquement acceptables, pour une utilisation selon l'une quelconque des revendications 1 à 12.
